(19) Europäisches Patentamt
European Patent Office
Office européen des brevets

(11) Veröffentlichungsnummer: **0 432 183 B1**

(12) # EUROPÄISCHE PATENTSCHRIFT

(45) Veröffentlichungstag der Patentschrift: **27.10.93**

(51) Int. Cl.$^5$: **C07H 19/10**, A61K 31/70

(21) Anmeldenummer: **89909191.2**

(22) Anmeldetag: **10.08.89**

(86) Internationale Anmeldenummer:
**PCT/EP89/00945**

(87) Internationale Veröffentlichungsnummer:
**WO 90/02134 (08.03.90 90/06)**

(54) **NEUE CYTIDIN-5'-DIPHOSPHATALKANOLE UND- GLYCEROLE, EIN VERFAHREN ZU DEREN HERSTELLUNG, SOWIE DEREN VERWENDUNG.**

(30) Priorität: **17.08.88 DD 319024**
**02.01.89 DD 324771**
**02.01.89 DD 324772**
**02.01.89 DD 324774**

(43) Veröffentlichungstag der Anmeldung:
**19.06.91 Patentblatt 91/25**

(45) Bekanntmachung des Hinweises auf die Patenterteilung:
**27.10.93 Patentblatt 93/43**

(84) Benannte Vertragsstaaten:
**AT BE CH DE FR GB IT LI LU NL SE**

(56) Entgegenhaltungen:
**GB-A- 2 168 350**
**US-A- 4 291 024**

(73) Patentinhaber: **Hafslund Nycomed Pharma Aktiengesellschaft**
**St. Peter-Strasse 25**
**A-4021 Linz(AT)**

(72) Erfinder: **BRACHWITZ, Hans**
**Wichertstrasse 9**
**O-1071 Berlin(DE)**
Erfinder: **LANGEN, Peter**
**Karowerchaussee 219**
**O-1115 Berlin(DE)**
Erfinder: **PALTAUF, Friedrich**
**St. Peter Hauptstrasse 29b/7**
**A-8036 Graz(AT)**
Erfinder: **HERMETTER, Albin**
**Sandgasse 25**
**A-8036 Graz(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Chemical Abstracts, vol. 74, 1971 (Columbus, Ohio, US) M. Kates et al.: "Synthesis of diphytanyl ether analogs of phosphatidic acid and cytidine diphosphate diglyceride", page 422, abstract No. 1122t & Can.J. Biochem. 1971, 49(3), 275-81

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 860 820**
**D-81635 München (DE)**

**Beschreibung**

Die Erfindung betrifft neue Cytidin-5'-diphosphatalkanole und -glycerole, ein Verfahren zu deren Herstellung, sowie deren Verwendung.

Es sind bereits Cytidindiphosphatdialkylglycerole in analoger Weise von M. Kates et al, Can J. Biochem. 49 (1971), 275 hergestellt worden. Diese Verbindungen sind natürlich vorkommend, sie sind beispielsweise Substrate der Phosphatidylinositolsynthase (E.C.2.6.1.67) und reagieren in Gegenwart von Inositol intrazellulär zu Phosphatidylinositol (R.H.Rao, Biochem. Biophysics Acta, 348, (1974), 306). Die Verbindungen zeigen keinerlei cytostatische Wirksamkeit. Addukte des Cytidinarabinosids (Ara-C), z B.: Ara-C-i-palmitoylglycerol (Chung IL Hong et al. J. Med. Chem., 31, 1988, 1793), sowie Ara-C-acylalkylglycerole (Chung Il Hong et al. ebenda, 29, 1986, 20 39) sind ebenfalls bekannt. Sie sind cytostatisch wirksam, wobei die Wirksamkeit auf die Anwesenheit des Ara-C zurückzuführen ist, das selbst ein starkes Cytostatikum darstellt. Ara-C-acyl- bzw. alkly-glyceroladdukte lassen sich als Depotform des Ara-C auffassen.

Es konnten nun neue Cytidin-5'-diphosphatalkanole und -glycerole gefunden werden, die ausgezeichnete cytostatische Wirkung aufweisen.

Gegenstand der Erfindung sind demnach Cytidin-5'-diphosphat-alkanole und - glycerole der allgemeinen Formel

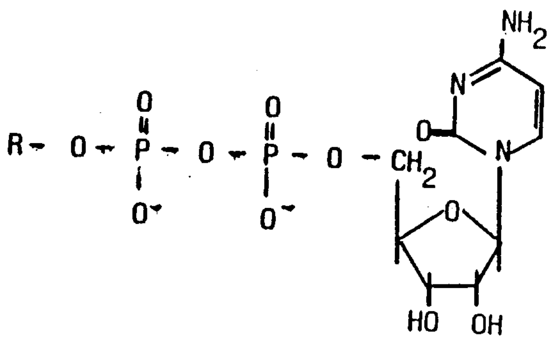

I

in der R einen gesättigten oder ungesättigten geradkettigen oder verzweigten, gegebenenfalls durch Halogen, Hydroxy, Alkoxy oder Cyano substituierten aliphatischen Rest mit 6 - 30 C-Atomen oder die Reste der allgemeinen Formeln

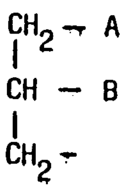

II,

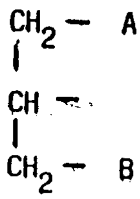

III

oder

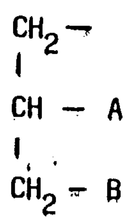

IV

bedeutet, wobei

- A einen gesättigten oder ungesättigten, geradkettigen oder verzweigten, gegebenenfalls durch Halogen, Hydroxy, Alkoxy oder Cyano substituierten Alkoxyrest mit 6 - 30 C-Atomen und
- B Wasserstoff, Halogen oder die Reste $-OCH_3$ oder $-O-(CH_2)_nCF_3$, wobei
- n die Zahlen 1 bis 3 bedeutet.

In der Formel I bedeutet R einen gesättigten oder ungesättigten, geradkettigen oder verzweigten aliphatischen Kohlenwasserstoffrest mit 6 - 30 C-Atomen. Vorzugsweise bedeutet R einen gesättigten oder ungesättigten, geradkettigen oder verzweigten Kohlenwasserstoffrest mit 10 - 20 C-Atomen. Beispiele für solche Reste sind Dodecyl-, Tetra-, Penta-, Hexa-, Hepta-, Octa-, Nonadecyl-, Eicosylreste bzw. deren verzweigte Analoga, oder Tetra-, Penta-, Hexa-, Hepta-, Octa-, Nonadecylen oder Eicosylenreste.

Diese Reste können gegebenenfalls auch substituiert sein, durch Halogen, beispielsweise Chlor, Brom, Iod oder Fluor, oder durch Hydroxy, Alkoxy oder Cyano.

Weiter kann R die Reste der allgemeinen Formeln II, III oder IV, wie vorstehend angegeben, bedeuten.

In den Formeln II, III und IV bedeutet A einen geradkettigen oder verzweigten oder Verzweigten, gesättigten oder ungesättigten Alkoxyrest mit 6 - 30 C-Atomen, Vorzugsweise 10 - 20 C-Atomen. Beispiele für solche Alkoxyreste sind Tetra-, Penta-, Hexa-, Hepta-, Octa-, Nonadecyloxy, Eicosyloxyreste, bzw. deren Verzweigte Analoge oder Tetra-, Penta-, Hexa-, Hepta-, Octa-, Nona- Dodecadecylenoxy, Eicosylenoxyreste. Diese Reste können gegebenenfalls auch substituiert sein, und zwar durch Halogen (Cl, Br, J oder F) oder Hydroxy, Alkoxy oder Cyano.

B bedeutet Wasserstoff oder Halogen, beispielsweise Chlor, Brom, Iod, Fluor, Vorzugsweise Chlor oder die Reste $-OCH_3$ oder $-O-(CH_2)_nCF_3$, wobei n die Zahlen 1 bis 3, Vorzugsweise die Zahl 1 bedeutet.

Besonders bevorzugte Einzelverbindungen sind

Cytidin-5'-diphosphat-hexadecanol

Cytidin-5'-diphosphat-dodecanol

Cytidin-5'-diphosphat-2-chlor-2 desoxy-1-0-hexadecylglycerol

Cytidin-5'-diphosphat-1-0-hexadecyl-2-0-(2,2,2-trifluorethyl)glycerol

Cytidin-5'-diphosphat-3-chlor-3-desoxy-1-0-hexadecylglycerol.

Cytidin-5'-diphosphat-1-0-hexadecyl-2-desoxyglycerol

Cytidin-5'-diphosphat-1-0-octadecyl-2-0-methylgycerol

Die neuen Cytidin-5'-diphosphat-alkanole und -glycerole können hergestellt werden, indem man Alkylphosphorsäuren bzw. Alklglycerophosphorsäuren der allgemeinen Formel

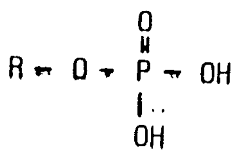

V

in der R die oben angegebene Bedeutung hat, mit Cytidin-5'-phosphat oder einem aktivierten Derivat dieser Verbindung in Gegenwart eines Kondensationsmittels oder einer organischen Base unter wasserfreien Bedingungen umsetzt.

Als aktiviertes Derivat des Cytidin-5'-phosphats kann beispielsweise Cytidin-5'-morpholidophosphat eingesetzt werden. Es ist zweckmäßig, einen der Reaktionspartner in Überschuß einzusetzten, wobei das Molverhältnis Cytidin-5'-phosphat zu Alkylphosphorsäure bzw. Alkylglycerophosphorsäure 1 : 0,5 bis 1 : 2 betragen kann.

Als Kondensationsmittel kann beispielsweise Dicylohexylcarbodiimid verwendet werden.

Als organische Basen werden beispielsweise 4-Dimethylaminopyridin oder Pyridin eingesetzt, wobei bei Verwendung von Pyridin die Umsetzung ohne Zusatz eines organischen Lösungsmittels erfolgen kann.

Gegebenenfalls kann als Lösungsmittel ein inertes organisches Lösungsmittel, beispielsweise Chloroform, eingesetzt werden.

Die Reaktion erfolgt bei Temperaturen Von etwa 20 - 60 °C, Vorzugsweise 35 - 40 °C.

Nach beendeter Reaktion wird das Produkt aus dem Reaktionsgemisch, beispielsweise durch Extraktion isoliert. Gegebenenfalls können die so erhaltenen Verbindungen, beispielsweise chromatographisch weiter gereinigt werden.

Die neuen Cytidin-5'-diphosphatalkanole und -glycerole zeichnen sich durch hohe cytostatische Wirksamkeit aus.

Zur Bestimmung der cytostatischen Wirkung wurde die antiproliferative Wirkung bei Ehrlich-Ascites Tumorzellen untersucht. Dabei zeigte sich, daß die erfindungsgemäßen Verbindungen ausgezeichnete cytostatische Wirkung aufweisen. Sie können daher allein oder in Mischung mit anderen Wirksubstanzen in Form üblicher galenischer Zubereitungen als Cytostatika oder Virustatika eingesetzt werden.

Die Verbindungen der allgemeinen Formel I sind zur Verwendung an Menschen bestimmt und können auf übliche Weise, beispielsweise oral oder parenteral verabreicht werden. Vorzugsweise werden sie oral verabreicht, wobei die Tagesdosis etwa 0,05 bis 20 mg/kg Körpergewicht beträgt, vorzugsweise 0,05 - 5 mg/kg Körpergewicht. Der behandelnde Arzt kann jedoch abhängig Vom Allgemeinzustand und dem Alter des Patienten, der entsprechenden Verbindung der allgemeinen Formel I der Art der Krankheit und der Art der Formulierung auch niedrigere oder höhere Dosen außerhalb dieses Bereiches verschreiben.

Die Verbindungen der Formel I können allein oder in Verbindung mit anderen pharmazeutisch aktiven Substanzen verabreicht werden, wobei der Gehalt der Verbindungen der allgemeinen Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, z. B. pharmazeutisch unbedenklichen Lösungsmitteln, Gelatine, Gummi arabicum, Milchzucker, Stärke, Magnesiumstearat, Talk, pflanzliche

Öle, Polyalkylenglykol, Vaseline oder dgl. vor. Die pharmazeutischen Präparate können in fester Form, beispielsweise als Tabletten, Dragees, Suppositorien, Kapseln u. dgl., in halbfester Form beispielsweise als Salben oder in flüssiger Form, beispielsweise als Suspensionen oder Emulsionen vorliegen. Gegenenfalls sind sie sterilisiert und enthalten Hilfsstoffe wie Konservierungs-, Stabilisierungs-und Emulgiermittel, Salz zur Veränderung des osmotischen Drucks u. dgl. Insbesondere können pharmazeutische Präparate die erfindungsgemäßen Verbindungen in Kombination mit anderen therapeutisch wertvollen Stoff enthalten. Mit diesen können die erfindungsgemäßen Verbindungen beispielsweise zusammen mit den oben genannten Hilfs-und/oder Trägerstoffe oder Verdünnungsmitteln zu Kombinationspräparaten formuliert werden.

Beispiel 1:

Cytidin-5'-diphosphat-hexadekanol

Hexadecylphosphat (150 mg, 0,47 mmol) wird viermal in ca. 5 ml Pyridin (über $CaH_2$ getrocknet) aufgenommen und die Lösung jeweils im Vakuum zur Trockne eingeengt.
Cytidin-5'-morpholidophosphat (4-Morpholin-N,N'-dicyclohexyl-carboxamidin-Salz) (404,2 mg, 0,58 mmol) wird viermal mit 4 ml Toluen versetzt und das Gemisch jeweils im Vakuum zur Trockne einrotiert. Danach werden die Reaktionskomponenten vereinigt, erneut mit 5 ml Pyridin einrotiert und in ca. 10 ml Pyridin aufgenommen. Die Lösung wird 5 Tage bei 37°C unter Ausschluß von Luftfeuchtigkeit gerührt. Anschließend wird das Reaktionsgemisch im Vakuum zur Trockne eingeengt. Zum Rückstand wird Toluen (3 x 5 ml) gegeben, das jeweils im Vakuum abdestilliert wird. Die erhaltene Substanz wird in einem Gemisch aus 15 ml Chloroform-Methanol (1 : 1, v/v) und 7 ml 0,01 N HCl aufgenommen. Die wäßrige Phase wird abgetrennt und dreimal mit etwa 5 ml Chloroform-Methanol extrahiert. Die vereinigten organischen Phasen werden mit wenig Wasser neutral gewaschen und zur Trockene eingeengt. Der Rückstand wird in einem Gemisch aus 45 ml Chloroform, 20 ml Methanol, 9 ml Wasser, 0,3 ml 1 N methanolischen Ammoniak behandelt, die organische Phase wird abgetrennt, nochmals mit 5 ml Wasser extrahiert. Durch Einengen der vereinigten wäßrigen Phasen wird die Verbindung I als Diammoniumsalz erhalten, welches zur weiteren Reinigung aus Chloroform-Aceton umgefällt wird. Die Ausbeute beträgt 144 mg Dünnschichtchloratographie (Merck Fertigplatte Kieselgel 60 F254): $R_f$ 0,31 (Chloroform-Methanol-Eisessig-Wasser, 25 : 15 : 2 : 4, v/v/v/v). UV-Absorption: $\lambda_{max}$ 272,6 nm (Wasser), $\lambda_{max}$282 (0,01 N HCl).

Beispiel 2:

Cytidin-5'-diphosphat-1-0-hexadecyl-2-0-(2,2,2-trifluorethyl)glycerol

Eine Mischung von 1-0-Hexadecyl-2-0-(2,2,2-trifluorethyl)glycerophosphorsäure (0,12 g, 0,25 mmol) und Cytidin-5'-morpholidophosphat (4-Morpholin-N,N'-dicyclohexylcarboxamidin-Salz) (0,30 g, 0,44 mmol) wird viermal mit je 5 ml Pyridin (mit $CaH_2$ getrocknet) im Hochvakuum zu Trockne einrotiert. Der Rückstand wird dann erneut in 6 ml Pyridin aufgenommen und die Lösung unter Ausschluß von Feuchtigkeit 9 Tage bei 37°C gerührt. Danach wird das Reaktionsgemisch im Vakuum zur Trockne eingeengt, der Rückstand mehrfach mit ca. 4 ml Toluen versetzt und erneut zur Trockne gebracht. Die erhaltene Substanz wird in einem Gemisch aus Chloroform-Methanol (1 : 1, v/v, 30 ml) und 0,01 N HCl (15 ml) aufgenommen. Durch tropfenweise Zugabe von 1 N HCl wird die Mischung auf einen pH-Wert Von 2 bis 3 eingestellt. Die Chloroform-Methanol-Phase wird abgetrennt, mit Wasser neutral gewaschen und nach Filtrieren eingeengt. Der Rückstand wird in einem Gemisch aus Chloroform (13 ml), Methanol-Wasser (2 : 1, v/v, 10 ml) 1 N methanol. $NH_3$ zweimal mit dem genannten Methanol-Wassermethanol. $NH_3$-Gemisch (ca. je 8 ml) extrahiert. Die vereinigten wäßrigen Phasen werden eingeengt. Der Rückstand wird mit wenig Methanol zur Kristallisation gebracht und anschließend mit trocknem Aceton mehrfach ausgewaschen. Nach dem Trocknen werden 30 mg des Diammoniumsalzes von I als chromatographisch einheitliche Substanz erhalten.
Dünnschichtchromatographie (Fertigplatte Kieselgel 60, Merck): Rf 0,39 (n-Propanol/25 %ig, wäßr. $NH_3$/Wasser, 8 : 2 : 1, v/v/v; Rf 0,30 (Chloroform/Methanol/Eisessig/Wasser, 25 : 15 : 2 : 4, v/v/v/v). UV-Absorption: $\lambda_{max}$ 275,5 nm (Wasser); λ. $_{max}$ 282 nm (0,01 N HCl).

Fast atom bombardment mass spectrometry:
m/z 783(M)

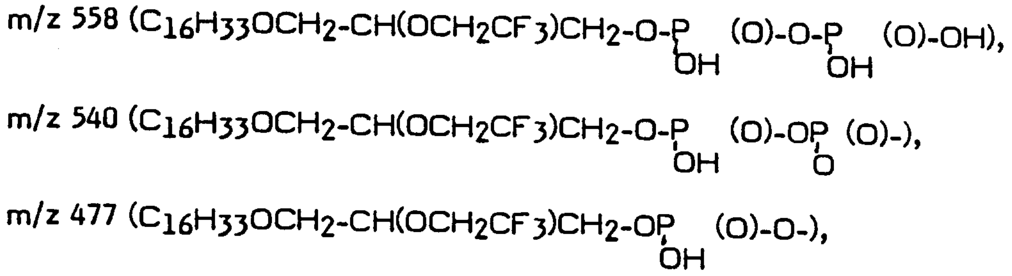

m/z 384(M-2H($C_{16}H_{33}OCH_2$-CH($OCH_2CF_3$)$CH_2$-0-))

Beispiel 3:

Cytidin-5'-diphosphat-3-chlor-3-desoxy-1-0-hexadecylglycerol

Zu einer Lösung von 0,664 g (1,6 mmol) 3-Chlor-3-desoxy-1-0-hexadecylglcero-2-phosphorsäure und 0,77 g (6,3 mmol) 4-Dimethylaminopyridin in 10 ml getrocknetem Chloroform werden 0,882 (1,3 mmol) Cytidin-5'-morpholidophosphat (4-Morpholin-N-N',-dicyclohexylcarboxamidin-Salz) gegeben. Die Mischung wird in einem verschlossenem Gefäß bei 35 °C unter Ausschluß von Feuchtigkeit 5 Tage gerührt. Danach wird das Reaktionsgemisch in Chloroform-Methanol-Wasser (pH-Wert 2) aufgenommen, die organische Phase mit Wasser gewaschen und im Vakuum einrotiert. Der Rückstand wird in Chloroform-Pyridin-Ameisensäure (50 : 30 : 7, v/v/v) aufgenommen und an 30 g Kieselgel 60 /Merck, 0,040 -0,063 mm) säulenchromatographisch getrennt. Die Elution erfolgt zunächst mit 270 ml des gleichen Lösungsmittels, danach mit Chloroform-Methanol (9 : 1, 340 ml; 8 : 2, 300 ml; jeweils v/v).
Die Fraktionen 31 - 55 (Fraktionsgröße 20 ml) werden im Vakuum zur Trockne eingeengt. Der erhaltene Rückstand wird in einem Gemisch aus 28 ml Chloroform-Methanol (1 : 1, v/v) und 9 ml 0,01 N HCl aufgenommen und die Mischung mit 1 N HCl auf den pH-Wert von 2 eingestellt. Die Phasen werden getrennt, die organische Phase wird nochmals mit 5 ml Chloroform-Methanol (1 : 1, v/v) behandelt. Die vereinigten organischen Phasen werden unter Zusatz von methanolischem Ammoniak einrotiert. Das erhaltene Diammoniumsalz der Verbindung I wird durch Behandlung mit Methanol-Diethylether zur Kristallisation gebracht, mit Diethylether gewaschen und getrocknet. Ausbeute: 0,340 g. Dünnschichtchromatographie (Merck Fertigplatte Kieselgel 60 F 254): Rf 0,42 (Chloroform-Methanol-Eisessig-Wasser 50 : 30 : 4 : 8, v/v/v/v); Rf 0,42 (n-Propanol-25 %wäßr. Ammoniak-Wasser 8 : 2 : 1, v/v/v).
UV-Absorption: $\lambda_{max}$ 281,5 nm (0,01 N HCl).

Beispiel 4:

Cytidin-5'-diphosphat-2-chlor-2-desoxy-1-0-hexadecylglycerol

Eine Mischung, bestehend aus 664 mg 2-Chlor-2-desoxy-1-0-hexadecylglycero-3-phosphorsäure, 882 mg 5'-Cytidinmorpholidophosphat (4-Morpholin-N,N'-dicyclohexylcarboxyamidin-Salz), 770 mg 4-Dimethylaminopyridin, wird unter wasserfreien Bedingungen in 30 ml getrocknetem Chloroform (alkoholfrei) 5 Tage bei 35 °C gerührt, nach beendeter Reaktion wurden zum Reaktionsgemisch Chloroform, Methanol, 0,02 % Salzsäure (je 20 ml) gegeben. Die wäßrige Phase wird mit 25 %iger Salzsäure auf pH 3 gebracht. Die untere Phase wird abgetrennt, mit Wasser gewaschen, getrocknet und im Vakuum zur Trockene eingeengt. Der Rückstand wird in 3 - 5 ml Chloroform/Pyridin/Ameisensäure 50 : 30 : 7, v/v/v, gelöst und in 25 g Kieselgel 60 (Merck, 0,04. - 0,063 mm) chromatographiert. Es wird zunächst mit 200 ml Chloroform/Methanol/Ameisensäure eluiert, danach mit Chloroform/Methanol (jeweils v/v) 9 : 2(1,1 L), 8 : 2-(0,5 L), 7,5 : 2,5(0,7 L), 6,5 : 3,5(0,3 L) und 6 : 4(2 L). Aus den Cloroform/Methanol-Fraktionen 70 - 96 (Fraktionsgröße 50 ml) werden 200 mg der Verbindung I erhalten. Die Substanz wird anschließend mit Chloroform/Methanol/2,5 n Salzsäure, 2 : 2 : 2, v/v/v, behandelt und die wäßrige Phase auf pH 2,5 eingestellt. Nach Abtrennung der unteren Phase wird die wäßrige Phase nochmals mit Chloroform/Methanol, 2 : 1, v/v, behandelt. Die vereinigten unteren Phasen wurden nach Zugabe von methanolschen Ammoniak einrotiert und so das Ammoniumsalz der Titelverbindung erhalten.
DC (Fertigplatte Kieselgel 60 F 250, Merck), Rf 0,33 ($CHCl_3/CH_3OH$, $CH_3$-COOH, $H_2O$, 29 : 18 : 2: v/v/v/v),
UV-Absorption: $\lambda_{max}$ 275,5 (neutral, $H_2O$), $\lambda_{max}$ 203,6(0,02 N HCl)

Beispiel A

Konzentrationsabhängige Wachstumshemmung von Ehrlich Ascites-Tumorzellen.

| Konzentration (µmolar) | 3 | 10 | 30 | 100 |
|---|---|---|---|---|
| Hemmung (%) | | | | |
| Verbindung nach Bsp. 1 | 12 | 19 | 46 | 31 |
| Verbindung nach Bsp. 2 | 9,5 | 30 | 86 | 100 |
| Verbindung nach Bsp. 3 | 12 | 17 | 29 | 69 |
| Verbindung nach Bsp. 4 | 18 | 38 | 67 | 94 |

**Patentansprüche**

**1.** Cytidin-5'-diphosphat-alkanole und -glycerole der allgemeinen Formel I

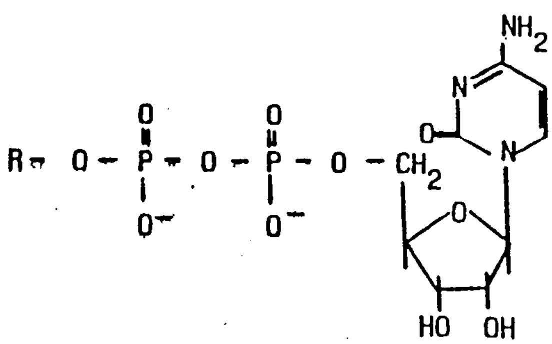

I

in der R einen gesättigten oder ungesättigten geradkettigen oder verzweigten, gegebenenfalls durch Halogen, Hydroxy, Alkoxy oder Cyano substituierten aliphatischen Rest mit 6 - 30 C-Atomen oder die Reste

| | | | | | |
|---|---|---|---|---|---|
| $CH_2$ - A | | $CH_2$ - A | | $CH_2$ - | |
| CH - B | II, | CH - | III, | CH - A | IV, |
| $CH_2$ - | | $CH_2$ - B | | $CH_2$ - B | |

bedeutete wobei

A einen gesättigten oder ungesättigten, geradkettigen oder verzweigten, gegebenenfalls durch Halogen, Hydroxy, Alkoxy oder Cyano substituierten Alkoxyrest mit 6 - 30 C-Atomen

und

B Wasserstoff, Halogen oder die Reste $-OCH_3$ oder $-O-(CH_2)_nCF_3$, wobei

n die Zahlen 1 bis 3 bedeutet.

**2.** Cytidin-5'-diphosphat-alkanole und -glycerole der allgemeinen Formel I nach Anspruch 1, in der R einen gesättigten oder ungesättigten, geradkettigen oder verzweigten, gegebenenfalls durch Halogen, Hydroxy, Alkoxy oder Cyano substituierten aliphatischen Rest mit 10 - 20 C-Atomen bedeutet oder einen der Reste der allgemeinen Formel II, III oder IV nach Anspruch 1, in dem A einen gesättigten oder ungesättigten, geradkettigen oder verzweigten Alkylrest mit 10 - 20 C-Atomen und B Chlor oder die Reste $-OCH_3$ oder $-OCH_2CF_3$ bedeutet.

**3.** Cytidin-5'--diphosphatalkanole und -glycerole nach Anspruch 1, ausgewählt aus der Gruppe
Cytidin-5'-diphosphatdodecanol

Cytidin-5'-diphosphathexadecanol
Cytidin-5'-diphosphat-2-chlor-2-desoxyhexadecylglycerol
Cytidin-5'-diphosphat-1-0-hexadecyl-2-0-(2,2,2,-trifluorethyl)glycerol
Cytidin-5'-diphosphat-3-chlor-3-desoxy-1-0-hexadecylglcerol
Cytidin-5'-diphosphat-1-0-hexadecyl-2-0-methylglycerol
Cytidin-5'-diphosphat-1-0-hexadecyl-2-desoxyglycerol

**4.** Verfahren zur Herstellung von Cytidin-5'-diphosphatalkanolen und - glycerolen; dadurch gekennzeichnet, daß man Alkylphosphorsäuren bzw. Alkylglycerophosphorsäuren der allgemeinen Formel V

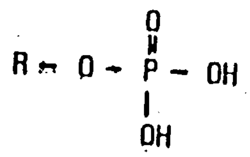

V

in der R die in Anspruch 1 angegebene Bedeutung hat, mit Cytidin-5'-phosphat oder einem aktivierten Derivat dieser Verbindung in Gegenwart eines Kondensationsmittels oder einer organischen Base unter wasserfreien Bedingungen umsetzt.

**5.** Verfahren nach Anspruch 7, dadurch gekennzeichnet, daß als Kondensationsmittel Dicyclohexylcarbodiimid oder als organische Base 4-Dimethylaminopyridin oder Pyridin eingesetzt wird.

**6.** Pharmazeutische Mittel, enthaltend Verbindungen der allgemeinen Formel I nach Anspruch 1, in Kombination mit üblichen galenischen Hilfs-, Träger- und/oder Verdünnungsmittel.

**7.** Pharmazeutische Mittel enthaltend Verbindungen der allgemeinen Formel I nach Anspruch 1, in Kombination mit anderen therapeutischen Wirkstoffen sowie üblichen galenischen Hilfs-, Träger- und/oder Verdünnungsmitteln.

## Claims

**1.** Cytidine-5'-diphosphate-alkanols and -glycerols of the general formula I

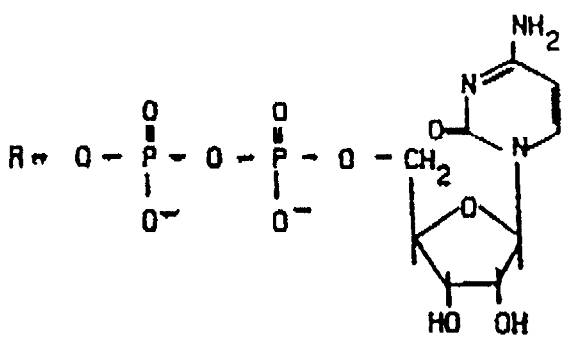

I

in which R denotes a saturated or unsaturated straight-chain or branched aliphatic radical having 6 - 30 C atoms, optionally substituted by halogen, hydroxyl, alkoxy or cyano or the radicals

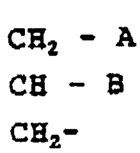 II, 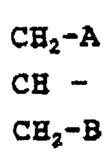 III, 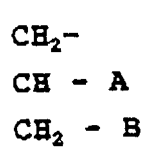 IV,

in which

A denotes a saturated or unsaturated, straight-chain or branched alkoxy radical having 6 - 30 C

atoms, optionally substituted by halogen, hydroxyl, alkoxy or cyano

and

B denotes hydrogen, halogen or the radicals $-OCH_3$ or $-O-(CH_2)_nCF_3$, in which

n denotes the numbers 1 to 3.

**2.** Cytidine-5'-diphosphate-alkanols and -glycerols of the general formula I according to Claim 1, in which R denotes a saturated or unsaturated, straight-chain or branched aliphatic radical having 10 - 20 C atoms, which is optionally substituted by halogen, hydroxyl, alkoxy or cyano, or one of the radicals of the general formula II, III or IV according to Claim 1, in which A denotes a saturated or unsaturated, straight-chain or branched alkyl radical having 10 - 20 C atoms and B denotes chlorine or the radicals $-OCH_3$ or $-OCH_2CF_3$.

**3.** Cytidine-5'-diphosphate-alkanols and -glycerols according to Claim 1, selected from the group comprising

cytidine-5'-diphosphate-dodecanol
cytidine-5'-diphosphate-hexadecanol
cytidine-5'-diphosphate-2-chloro-2-desoxyhexadecylglycerol
cytidine-5'-diphosphate-1-O-hexadecyl-2-O-(2,2,2-trifluoroethyl)glycerol
oytidine-5'-diphosphate-3-chloro-3-desoxy-1-O-hexadecylglycerol,
cytidine-5'-diphosphate-1-O-hexadecyl-2-O-methylglycerol
cytidine-5'-diphosphate-1-O-hexadecyl-2-desoxyglycerol

**4.** Process for the preparation of cytidine-5'-diphosphate-alkanols and -glycerols, characterised in that alkylphosphoric acids or alkylglycerophosphoric acids of the general formula V

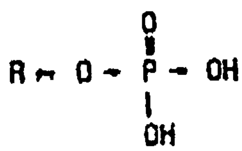

V

in which R has the meaning indicated in Claim 1, are reacted under anhydrous conditions with cytidine-5'-phosphate or an activated derivative of this compound in the presence of a condensing agent or an organic base.

**5.** Process according to Claim 4, characterised in that dicyclohexylcarbodiimide is employed as the condensing agent or 4-dimethylaminopyridine or pyridine is employed as the organic base.

**6.** Pharmaceutical compositions, containing compounds of the general formula I according to Claim 1, in combination with customary pharmaceutical auxiliaries, excipients and/or diluents.

**7.** Pharmaceutical compositions, containing compounds of the general formula I according to Claim 1, in combination with other therapeutic active compounds and customary pharmaceutical auxiliaries, excipients and/or diluents.

## Revendications

**1.** Alcanols et glycérols de cytidine-5'-diphosphate répondant à la formule générale I

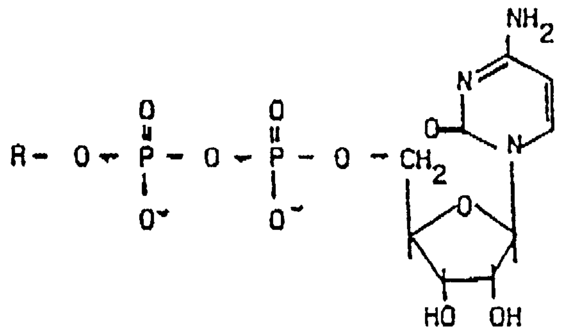

(I)

dans laquelle R signifie un reste aliphatique contenant de 6 à 30 atomes de carbone, saturé ou insaturé, linéaire ou ramifié, substitué éventuellement par halogène, hydroxy, alcoxy ou cyano, ou les restes répondant aux formules générales :

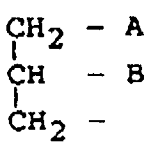

(II),

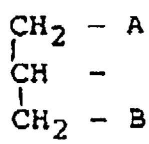

(III) ou

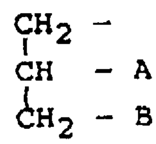

(IV)

dans lesquelles

A signifie un reste alcoxy contenant de 6 à 30 atomes de carbone, saturé ou insaturé, linéaire ou ramifié, substitué éventuellement par halogène, hydroxy, alcoxy ou cyano, et

B signifie l'hydrogène, un halogène ou les restes $-OCH_3$ ou $-O-(CH_2)_nCF_3$, où n signifie un chiffre valant de 1 à 3.

**2.** Alcanols et glycérols de cytidine-5'-diphosphate de formule générale I selon la revendication 1, où R signifie un reste aliphatique contenant de 10 à 20 atomes de carbone, saturé ou insaturé, linéaire ou ramifié, substitué éventuellement par halogène, hydroxy, alcoxy ou cyano, ou un des restes des formules générales II, III ou IV selon la revendication 1, où A signifie un reste alkyle contenant de 10 à 20 atomes de carbone, saturé ou insaturé, linéaire ou ramifié, et B signifie le chlore ou le reste $-OCH_3$ ou $-OCH_2CF_3$.

**3.** Alcanols et glycérols de cytidine-5'-diphosphate selon la revendication 1, choisis dans le groupe :
cytidine-5'-diphosphate-dodécanol,
cytidine-5'-diphosphate-hexadécanol,
cytidine-5'-diphosphate-2-chloro-2-désoxyhexadécylglycérol,
cytidine-5'-diphosphate-1-0-hexadécyl-2-0-(2,2,2-trifluoroéthyl)glycérol,
cytidine-5'-diphosphate-3-chloro-3-désoxy-1-0-hexadécylglycérol,
cytidine-5'-diphosphate-1-0-hexadécyl-2-0-méthylglycérol
cytidine-5'-diphosphate-1-0-hexadécyl-2-désoxyglycérol.

**4.** Procédé de préparation des alcanols et glycérols de cytidine-5'-diphosphate, caractérisé en ce qu'on fait réagir des acides alkylphosphoriques ou des acides alkylglycérophosphoriques répondant à la formule générale V

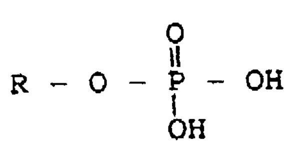

(V)

dans laquelle R a la signification indiquée dans la revendication 1, avec le cytidine-5'-phosphate ou un dérivé activé de ce composé, en présence d'un agent de condensation ou d'une base organique, dans des conditions anhydres.

**5.** Procédé selon la revendication 4, caractérisé en ce qu'on utilise, comme agent de condensation, le dicyclohexylcarbodiimide ou, comme base organique, la 4-diméthylaminopyridine ou la pyridine.

**6.** Substances pharmaceutiques contenant des composés de formule générale I selon la revendication 1, combinées à des adjuvants, excipients et/ou diluants galéniques usuels.

**7.** Substances pharmaceutiques contenant des composés de formule générale I selon la revendication 1, combinées à d'autres substances thérapeutiques, ainsi qu'à des adjuvants, excipients et/ou diluants galéniques usuels.